# EUROPÄISCHE PATENTSCHRIFT

(11) **EP 2 736 980 B1**
(45) Veröffentlichungstag und Bekanntmachung des Hinweises auf die Patenterteilung: **29.04.2015**
(21) Anmeldenummer: 12731435.9
(22) Anmeldetag: 29.06.2012
(51) Int. Cl.: C09D 4/00, C09J 4/00

(54) **NIEDERMOLEKULARE PRODUKTE UND DEREN VERWENDUNG ALS REVERSIBLE ODER PERMANENTE NIEDERTEMPERATUR-VERNETZER BEI DIELS-ALDER-REAKTIONEN**
LOW MOLECULAR WEIGHT PRODUCTS AND USE THEREOF AS REVERSIBLE OR PERMANENT LOW-TEMPERATURE CROSSLINKING AGENT IN DIELS-ALDER REACTIONS
PRODUITS À FAIBLE MASSE MOLÉCULAIRE ET LEUR UTILISATION COMME AGENTS DE RÉTICULATION À BASSE TEMPÉRATURE RÉVERSIBLES OU PERMANENTS DANS DES RÉACTIONS DE DIELS-ALDER

(30) Priorität: 29.07.2011 DE 102011080131
(43) Veröffentlichungstag der Anmeldung: 04.06.2014
(73) Patentinhaber: Evonik Degussa GmbH, 45128 Essen (DE)
(72) Erfinder: SCHMIDT, Friedrich, Georg, 45721 Haltern am See (DE); HILF, Stefan, 63517 Rodenbach (DE); SPYROU, Emmanouil, 46514 Schermbeck (DE); ZHOU, Jiawen, 47443 Moers (DE); GUIMARD, Nathalie, 66123 Saarbrücken (DE); BARNER-KOWOLLIK, Christopher, 76297 Stutensee (DE); ÖHLENSCHLÄGER, Kim Klaus, 68766 Hockenheim (DE); HENNIG, André, 55218 Ingelheim (DE)
(86) Internationale Anmeldenummer: PCT/EP2012/062683
(87) Internationale Veröffentlichungsnummer: WO 2013/017349

(56) Entgegenhaltungen:
- WO-A1-2012/065786
- WO-A2-2010/144774
- US-A1- 2010 099 798

## Beschreibung

Die Erfindung betrifft niedermolekulare Produkte, deren Herstellung und den Einsatz als reversible oder permanente Vernetzer in Polymeren bzw. Polymernetzwerken, wobei die Verknüpfung bzw. Vernetzung der entstehenden Polymere über Diels-Alder-Reaktionen bewirkt wird.

Methoden zur reversiblen Vernetzung von Polymeren sind für ein breites Feld von Anwendungen von großem Interesse. Beispielsweise in Klebanwendungen sind diverse Möglichkeiten für die Automobilindustrie oder die Halbleiterindustrie beschrieben. Aber auch bei der Konstruktion von Maschinen, feinmechanischen Geräten oder in der Bauindustrie sind solche Klebstoffe interessant.

Neben Klebanwendungen können reversibel vernetzbare Polymere auch in Dichtstoffen, Beschichtungsmassen wie Lacken oder Farben oder bei der Herstellung von Formkörpern z. B. über Rapid Prototyping - Verfahren interessant sein.

Als bekannteste Vernetzer-Moleküle für Diels-Alder-Vernetzungen sollen hier die bereits seit langem kommerziell erhältlichen Bismaleinimid-Bausteine (COMPIMIDE^{®} von Evonik AG) genannt sein.

Unter dem Oberbegriff "Click Chemie" werden seit einigen Jahren vor allem in der akademischen Welt Methoden zum Aufbau von Blockcopolymeren erforscht. Dabei werden zwei unterschiedliche Homopolymere mit verknüpfbaren Endgruppen miteinander kombiniert und z.B. mittels einer Diels-Alder-Reaktion, Diels-Alder-analogen Reaktion bzw. einer anderen Cycloaddition miteinander verbunden. Ziel dieser Reaktion ist es, thermisch stabile, lineare und ggf. hochmolekulare Polymerketten aufzubauen. In Inglis et al. (Macromolecules 2010, 43, S. 33 - 36) beispielsweise sind zu diesem Zweck Polymere mit Cyclopentadienylendgruppen, die aus mittels ATRP hergestellten Polymeren erhältlich sind, beschrieben. Diese Cyclopentadiengruppen können sehr rasch in Hetero-Diels-Alder Reaktionen mit Polymeren reagieren, welche elektronenarme Dithioester als Endgruppen tragen (Inglis et al. Angew. Chem. Int. Ed. 2009, 48, S. 2411 - 2414).

Die Verwendung von monofunktionellen RAFT-Polymeren zur Verknüpfung mit monofunktionellen Polymeren, die eine Dihydrothiopyran-Gruppe über eine Hetero-Diels-Alder-Reaktion findet sich in Sinnwell et al. (Chem. Comm. 2008, 2052-2054). Mit dieser Methode lassen sich AB-Diblockcopolymere realisieren. Schnelle Varianten dieser Hetero-Diels-Alder-Verknüpfung zur Synthese von AB-Blockcopolymeren mit einer nach einer RAFT-Polymerisation vorliegenden Dithioester-Gruppe und einer Dienyl-Endgruppe sind in Inglis et al. (Angew.Chem.Int.Ed. 2009,48, S. 2411-14) und in Inglis et al. (Macromol. Rapd Commun. 2009, 30, S. 1792-98) beschrieben. Die analoge Herstellung von Multiarm-Sternpolymeren findet sich in Sinnwell et al. (J.Pol.Sci.: Part A: Pol.Chem. 2009, 47, S. 2207-13).

In US 6,933,361 ist ein System zur Herstellung von einfach reparierbaren, transparenten Formkörpern beschrieben. Das System besteht aus zwei multifunktionellen Monomeren, die mittels einer Diels-Alder-Reaktion zu einem hochdichten Netzwerk polymerisieren. Dabei handelt es sich bei der einen Funktionalität um ein Maleinsäureimid und bei der anderen Funktionalität um ein Furan. Das thermische Schalten eines solchen hochdichten Netzwerks dient zur Reparatur desselbigen. Die Vernetzung findet bei Temperaturen oberhalb von 100 °C statt. Die partielle Rückreaktion bei noch höheren Temperaturen.

Bei Syrett et al. (Polym.Chem. 2010, DOI: 10.1039/b9py00316a) werden Sternpolymere zur Verwendung als Fließverbesserer in Ölen beschrieben. Diese weisen mittels einer reversiblen Diels-Alder-Reaktion steuerbare, selbstheilende Eigenschaften auf. Dazu werden monofunktionelle Polymethacrylatarme mit Polymethacrylaten kombiniert, die in der Mitte der Kette, als Fragment des eingesetzten Initiators über eine in einer reversiblen Diels-Alder-Reaktion verwendbare Gruppe verfügen.

In der Patentanmeldung DE102010001987.9 sind vernetzbare Systeme offenbart, die einen thermoreversiblen Vernetzungsmechanismus auf Basis einer Diels-Alder- oder einer Hetero-Diels-Alder-Reaktion aufweisen. In der DE102010001992.5 sind analoge Systeme offenbart, die mittels des gleichen thermoreversiblen Mechanismus eine steuerbare Viskosität aufweisen.

Die US 4,513,125 A offenbart eine Zusammensetzung für spezielle kathodische Elektro-Tauchlack-Beschichtungen, bei der ein Polydiene-funktionalisiertes Epoxy-Amin mit einem Polydienenophil-functionalisierten Polyisocyanat-Oligomer bei erhöhten Temperaturen miteinander reagieren. Die Polydienophil- functionalisierten Polyisocyanat-Oligomere haben eine Funktionalität von mindestens 3. Konkret erwähnt werden Furfuryl-Alkohol bzw. Furfurylamin, 2-Hydroxymethyl-1,3-butadiene, 2Aminomethyl-1,3-butadien oder Mischungen derselben. Sorbinalkohol-Derivate werden allerdings nicht erwähnt.

### Aufgabe

Aufgabe der vorliegenden Erfindung war es, leicht zu synthetisierende und vielseitig einsetzbare niedermolekulare Vernetzermoleküle für Diels-Alder-Reaktionen bei vorzugsweise niedrigen Temperaturen und mit der Möglichkeit einer retro-Diels-Alder-Reaktion für reversible Vernetzungen zu finden, welche zudem noch besonders ökologisch sind.

Die Aufgabe wurde durch die neuen Reaktionsprodukte der vorliegenden Erfindung gelöst.

Gegenstand der Erfindung sind Reaktionsprodukt aus
A) mindestens einem Isocyanat und/oder Amin mit mindestens zwei funktionellen Gruppen pro Molekül der allgemeinen Formel 1 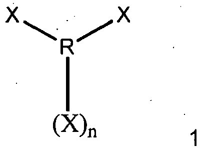 mit der Bedeutung
   - n=: 0, 1,
   - X=: NCO, NH₂, NHA,
   - A=: H, gleichzeitig oder unabhängig voneinander Alkylrest mit 1 bis 16 Kohlenstoffatomen, cyclischer Kohlenwasserstoffrest, aromatischer Kohlenwasserstoffrest, wobei dieser Rest auch Heteroatome und/oder funktionelle Gruppen und/oder Doppelbindungen enthalten kann,
   - R=: aliphatischer oder cycloaliphatischer Kohlenwasserstoffrest, der auch Heteroatome und/oder funktionelle Gruppen und/oder Doppelbindungen enthalten kann,
   und
B) mindestens einem Dien mit einer funktionellen Gruppe der allgemeinen Formel 2 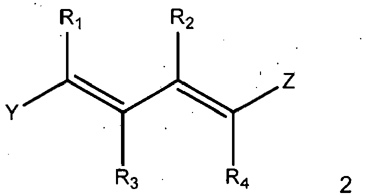 mit der Bedeutung
   - Y=: CH₂OH COOH, COOA, CH₂NH₂, CH₂NHA,
   - A, Z und R¹ - R⁴ =: gleichzeitig oder unabhängig von einander Alkylrest mit 1 bis 16 Kohlenstoffatomen, cyclischer Kohlenwasserstoffrest, aromatischer Kohlenwasserstoffrest, wobei diese Reste auch Heteroatome und/oder funktionelle Gruppen und/oder Doppelbindungen enthalten können,
wobei alle funktionellen Gruppen X von A) mit der äquivalenten Menge von B) umgesetzt sind.

Überraschend wurde gefunden, dass die Verbindungen gemäß der Erfindung, mit Dienophilen bereits bei Raumtemperatur oder nur leicht erhöhten Temperaturen vernetzbar sind und die Vernetzung bei einer höheren Temperatur zu mindestens 50 % wieder rückgängig gemacht werden kann.

Es wurde gefunden, dass diese Systeme bereits bei Raumtemperatur, gegebenenfalls unter Zugabe eines Vernetzungskatalysators, sehr schnell vernetzen. Genauso wurde gefunden, dass diese Netzwerke bereits bei sehr geringen Temperaturen von z.B. etwas über 80 °C wieder einfach und nahezu vollständig in einen Thermoplasten zurückgeführt werden können. Darüber hinaus wurde sehr überraschend gefunden, dass daraufhin eine erneute Vernetzung, ohne weitere Zugabe von Vernetzer und/oder Katalysator, z.B. durch reines Abkühlen wieder erfolgen kann. Darüber hinaus ist es ein besonderer Effekt, dass diese Zyklen aus Vernetzen und Rücküberführung in einen Thermoplasten mindestens dreimal, bevorzugt mindestens fünfmal ohne einen größeren Eigenschaftsverlust des Netzwerks durchgeführt werden können.

Als Isocyanate Komponente A) eignen sich aliphatische, cycloaliphatische und araliphatische, d. h. arylsubstituierte aliphatische Diisocyanate, wie sie beispielsweise im Houben-Weyl, Methoden der organischen Chemie, Band 14/2, Seiten 61 - 70 und im Artikel von W. Siefken, Justus Liebigs Annalen der Chemie 562, 75 - 136, beschrieben werden, wie 1,2-Ethylendiisocyanat, 1,4-Tetramethylendiisocyanat, 1,6-Hexamethylendiisocyanat (HDI), 2,2,4-Trimethyl-1,6-hexamethylendiisocyanat (TMDI), 2,4,4-Trimethyl-1,6-hexamethylendiisocyanat (TMDI) sowie deren Gemische, 1,9-Diisocyanato-5-methylnonan, 1,8-Diisocyanato-2,4-dimethyloctan, 1,12-Dodecandiisocyanat, ω,ω'-Diisocyanatodipropylether, Cyclobuten-1,3-diisocyanat, Cyclohexan-1,3-diisocyanat, Cyclohexan-1,4-diisocyanat, 3-Isocyanatomethyl-3,5,5-trimethyl-cyclohexylisocyanat (Isophorondiisocyanat, IPDI), 1,4-Diisocyanatomethyl-2,3,5,6-tetramethyl-cyclohexan, Decahydro-8-methyl-(1,4-methanol-naphthalin-2,5-ylendimethylendiisocyanat,, Decahydro-8-methyl-(1,4-methanol-naphthalin-3,5-ylendimethylendiisocyanat, Hexahydro-4,7-methano-indan-1,5-ylendimethylendiisocyanat, Hexahydro-4,7-methano-indan-2,5-ylendimethylendiisocyanat, Hexahydro-4,7-methano-indan-1,6- ylendimethylendiisocyanat, Hexahydro-4,7-methano-indan-2,5- ylendimethylendiisocyanat, Hexahydro-4,7-methanoindan-1,5-ylendiisocyanat, Hexahydro-4,7-methanoindan-2,5-ylendiisocyanat, Hexahydro-4,7-methanoindan-1,6-ylendiisocyanat, Hexahydro-4,7-methanoindan-2,6-ylendiisocyanat, 2,4-Hexahydrotoluylendiisocyanat, 2,6-Hexahydrotoluylendiisocyanat, 4,4'-Methylendicyclohexyldiisocyanat (4,4'-H₁₂MDI), 2,2'-Methylendicyclohexyl-diisocyanat (2,2'-H₁₂MDI), 2,4-Methylendicyclohexyldiisocyanat (2,4-H₁₂MDI) oder beliebige Mischungen dieser Isomere , 4,4'-Diisocyanato-3,3',5,5'-tetramethyldicyclohexylmethan, 4,4'-Diisocyanato-2,2',3,3',5,5',6,6'-octamethyldicyclohexylmethan, ω,ω'-Diisocyanato-1,4-diethylbenzol, 1,4-Diisocyanatomethyl-2,3,5,6-tetramethylbenzol, 2-Methyl-1,5-diisocyanatopentan (MPDI), 2-Ethyl-1,4-diisocyanatobutan, 1,10-Diisocyanatodecan, 1,5-Diisocyanatohexan, 1,3-Diisocyanatomethylcyclohexan, 1,4-Diisocyanatomethylcyclohexan sowie beliebige Gemische dieser Verbindungen. Weitere geeignete Isocyanate werden in dem genannten Artikel in den Annalen auf Seite 122 f. beschrieben. Auch 2,5-Bis(isocyanatomethyl)bicyclo[2.2.1]heptan (NBDI)und/oder (2,6)-Bis(isocyanatomethyl)bicyclo[2.2.1]heptan (NBDI) ist in Reinsubstanz oder als Mischkomponente geeignet. Die Herstellung dieser Diisocyanate wird heutzutage in der Regel entweder über die Phosgenroute oder über das Harnstoffverfahren durchgeführt. Die Produkte beider Methoden sind gleichermaßen für die Verwendung im erfindungsgemäßen Verfahren geeignet.

Besonders bevorzugt werden aliphatischen und cycloaliphatischen Diisocyanate eingesetzt. Ganz besonders bevorzugt werden IPDI, TMDI, HDI und/oder H₁₂MDI, eingesetzt, allein oder in Mischungen.

Eine andere bevorzugte Klasse von Polyisocyanaten als Komponente A) sind die durch Dimerisierung, Trimerisierung, Allophanatisierung, Biuretisierung und/oder Urethanisierung der einfachen Diisocyanate hergestellten Verbindungen mit mehr als zwei isocyanatgruppen pro Molekül, beispielsweise die Umsetzungsprodukte dieser einfachen Diisocyanate, wie z. B. IPDI, TMDI, HDI und/oder H₁₂MDI mit mehrwertigen Alkoholen (z. B. Glycerin, Trimethylolpropan, Pentaerythrit) bzw. mehrwertigen Polyaminen.

Besonders bevorzugt werden auch die Isocyanurate, die durch Trimerisierung der einfachen Diisocyanate erhältlich sind, eingesetzt. Ganz besonders bevorzugt werden die Trimeren von IPDI, HDI und/oder H₁₂MDI, eingesetzt, allein oder in Mischungen.

Als Komponente A) eignen sich auch aliphatische, cycloaliphatische und araliphatische, d. h. arylsubstituierte aliphatische Amine mit mindestens zwei Aminogruppen im Molekül.

Besonders bevorzugt sind Diamine ausgewählt aus 1,3- und 1,4-Diaminomethylcyclohexan, Hexandiamin-1,6 (HDA), 2,2,4- bzw. 2,4,4-Trimethylhexanamin-1,6 sowie deren Gemische, 3-Aminomethyl-3,5,5-trimethylcyclohexylamin (Isophorondiamin, IPDA), 4,4'-Methylendicyclohexyldiamin, 2,4-Methylendicyclohexyldiamin, 2,2'-Methylendicyclohexyldiamin sowie beliebige Mischungen dieser Isomeren (H₁₂MDA), Polyetherdiamine, eingesetzt werden. Es können auch Gemische der Amine eingesetzt werden.

Besonders bevorzugt sind IPDA, HDA und/oder H₁₂MDA.

Als Diene B) werden Diene mit nur einer funktionellen Gruppe eingesetzt, der allgemeinen Formel 2 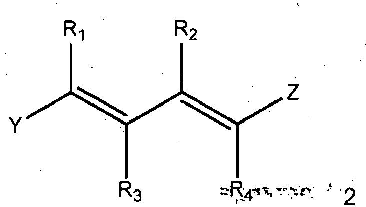 mit der Bedeutung
- Y=: CH₂OH, COOH, COOA, CH₂NH₂, CH₂NHA,
mit
- A, Z und R¹ - R⁴ =: gleichzeitig oder unabhängig von einander Alkylrest mit 1 bis 16 Kohlenstoffatomen, cyclischer Kohlenwasserstoffrest, aromatischer Kohlenwasserstoffrest, wobei diese Reste auch Heteroatome und/oder funktionelle Gruppen und/oder Doppelbindungen enthalten können.

Dabei handelt es sich bei dem Rest Z um einen inerten Rest, der nicht mit der Komponente A) reagiert.

Der Rest Y reagiert mit den funktionellen Gruppen X der Komponente A) zum Reaktionsprodukt.

Besonders bevorzugt wird Sorbinalkohol 3 und/oder Sorbinsäure 4 eingesetzt.

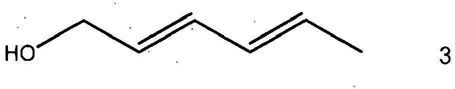

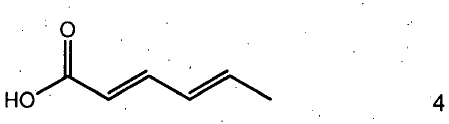

Eine weitere bevorzugte Klasse von Dienen B) sind die sogenannten Retinoide der folgenden Formel 5: 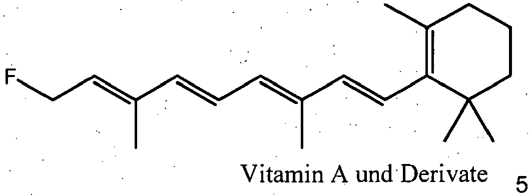 F = OH, COOH, COOK, CHO,
mit K gleich Alkylrest mit 1 bis 6 Kohlenstoffatomen, cyclischer Kohlenwasserstoffrest, aromatischer Kohlenwasserstoffrest, wobei dieser Rest auch Heteroatome und/oder funktionelle Gruppen und/oder Doppelbindungen enthalten kann,
bevorzugt der Formel 6:

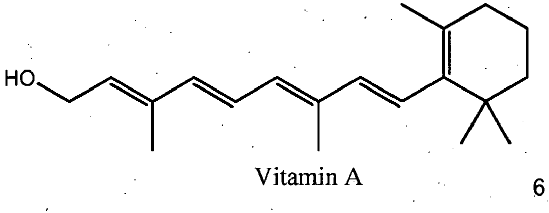

Es ist für den Fachmann verständlich, dass die funktionellen Gruppen der Verbindungen A) und B) so ausgewählt werden müssen, dass diese miteinander reagieren.

Als ein Beispiel für ein erfindungsgemäßes Reaktionsprodukt sei hier die Umsetzung von Sorbinalkohol mit Isophorondiisocyanat zu einem difunktionellen zur Diels-Alder-Reaktion fähigen Dien-Baustein aufgeführt:

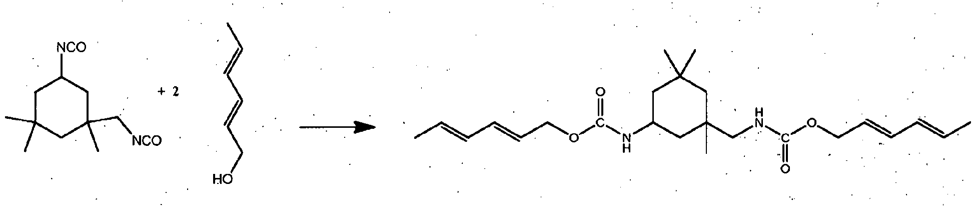

Als ein Beispiel für ein erfindungsgemäßes Reaktionsprodukt mit Trimeren - Isocyanurate sei hier die Umsetzung von Sorbinalkohol mit Isophoronisocyanurat zu einem trifunktionellen zur Diels-Alder-Reaktion fähigen Dien-Baustein aufgeführt:

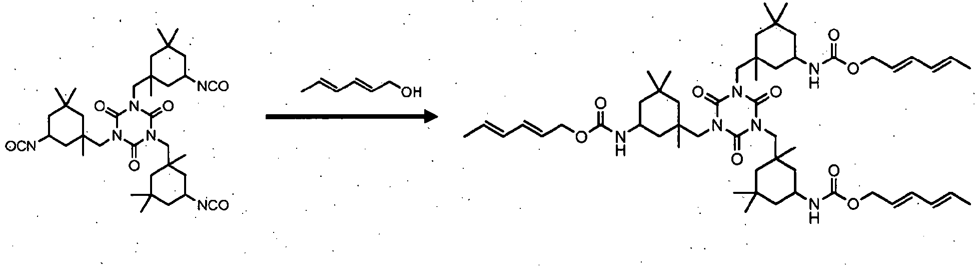

Die Diene können aus dem nachwachsenden Rohstoff Sorbitol hergestellt werden. Es handelt sich dabei um Sorbinderivate, die besonders ökologisch sind. Gleiches gilt für die Retinoide.

Ein weiterer Gegenstand der vorliegenden Erfindung ist die Verwendung der erfindungsgemäßen Verbindungen zur reversiblen oder permanenten Vernetzung mit Verbindungen mit mindestens einer reaktiven Doppelbindung, im Folgenden auch Dienophile genannt.

Als Dienophile werden solche Verbindungen definiert, die über eine reaktive Doppelbindung verfügen, wobei die Doppelbindung mittels einer Diels-Alder-Reaktion mit den beiden Doppelbindungen der erfindungsgemäßen Reaktionsprodukte reagieren kann. Diese Reaktion läuft auch in umgekehrter Richtung ab, wobei die einzelnen Komponenten wieder entstehen: Die Reaktion ist also reversibel und wird retro- Diels-Alder-Reaktion genannt. Dabei wird eine Formulierung aus zumindest zwei unterschiedlichen Komponenten, mittels einer Diels-Alder- bzw. einer Hetero-Diels-Alder-Reaktion bei Raumtemperatur vernetzt. In einem zweiten Verfahrensschritt werden bei einer höheren Temperatur mindestens 50 %, bevorzugt mindestens 90 % und besonders bevorzugt mindestens 99 % der Vernetzungsstellen mittels einer Retro-Diels-Alder- bzw. einer Retro-Hetero-Diels-Alder-Reaktion wieder gelöst.

In einer bevorzugten Ausführungsform handelt es sich bei der Dienophil-Komponente um ein mittels Atomtransfer radikalische Polymerisation (ATRP) hergestelltes, bifunktionelles Polymer. In diesem Fall erfolgt die Funktionalisierung mit den Diengruppen durch eine polymeranaloge oder während des Abbruchs durchgeführte Substitution endständiger Halogenatome. Diese Substitution kann beispielsweise durch Zugabe von mit Diengruppen funktionalisierten Mercaptanen erfolgen.

Bevorzugt handelt es sich bei dem Dienophil um eine Verbindung mit einer Kohlenstoff-Schwefel-Doppelbindung und damit bei der bevorzugten Vernetzungsreaktion um eine Hetero-Diels-Alder-Reaktion.

In der gleichen bevorzugten Ausführungsform kann als Dienophil-Komponente eine niedermolekulare organische Verbindung mit 3 bis 4 Dithioestergruppen, entsprechend der oberen Ausführung eine die Elektronendichte der C=S Doppelbindung stark verringernden Gruppe Z aufweisend, verwendet werden.

Besonders bevorzugt handelt es sich bei dem Dienophil um einen Dithioester.

Ganz besonders bevorzugt ist das Dienophil eine Verbindung mit der Struktur 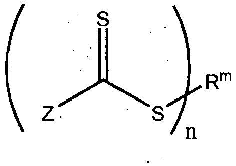 wobei es sich bei Z um eine - bevorzugt stark - Elektronen-ziehende Gruppe, bei R^{m} um eine mehrbindige organische Gruppe, bevorzugt basierend auf verzweigten oder linearen alkylischen, aromatischen oder einer Kombination aus alkylischen und aromatischen multifunktionalen Alkoholen, multifunktionalen halogenierten Verbindungen, multifunktionalen Carbonsäuren oder multifunktionalen Amine handelt. Alternativ kann es sich bei R^{m} auch um ein Polymer handeln. Die Anzahl der Dithioestergruppen n ist eine Zahl zwischen 2 und 20, bevorzugt zwischen 2 und 10 und besonders bevorzugt zwischen 2 und 4.

In einer bevorzugten Ausführungsform ist die Gruppe Z eine 2-Pyridylgruppe, eine Phosphorylgruppe oder eine Sulfphonylgruppe. Des Weiteren kommen in Frage Cyano- oder Trifluoromethylgruppen sowie jede andere Gruppe Z, welche die Elektronendichte der C=S Doppelbindung sehr stark verringert und somit eine rasche Diels-Alder-Reaktion erlaubt.

Die durch diese erfindungsgemäßen Vemetzer-Moleküle einfach zugänglichen reversibel oder permanent vernetzbaren Polymere sind für ein breites Feld von Anwendungen von großem Interesse. Beispielsweise für wieder lösbare Klebanwendungen sind dem Fachmann wohlbekannt diverse Möglichkeiten für die Automobilindustrie oder die Halbleiterindustrie beschrieben. Aber auch bei der Konstruktion von Maschinen, feinmechanischen Geräten oder in der Bauindustrie sind solche Klebstoffe interessant. Die erfindungsgemäßen Formulierungen bzw. Verfahren können in verschiedensten Anwendungsgebieten zur Verwendung kommen. Die nachfolgende Liste zeigt einige bevorzugte Anwendungsgebiete beispielhaft auf, ohne die Erfindung diesbezüglich in irgendeiner Form einzuschränken. Solche bevorzugten Anwendungsgebiete sind z. B. Klebstoffe, Formmassen, Tinten, Dichtstoffen, Beschichtungsmassen wie Lacken oder Farben, Verbundmaterialien oder die Verwendung bei der Herstellung von Formkörpern z. B. über Rapid-Prototyping-Verfahren.

Ein Beispiel für Anwendungen im Bereich Rapid-Prototyping für die hier beschriebenen vernetzenden und entnetzenden Materialien sind im Bereich FDM (Fused Deposition Modeling) oder beim 3D-Druck per Ink-jet-Verfahren mit niedrigviskosen Schmelzen zu finden.

Durch die nachfolgenden Beispiele wird die Erfindung sowie die Ausführbarkeit näher beschrieben.

### Beispiele

Herstellungsbeschreibungen der Sorbinalkohol-IPDI-Addukte bzw. Sorbinalkohöl-T1890-Addukte

### Beispiel 1

### IPDI-Sorbinalkohol-Addukt (IPDI = Isophorondiisocyanat) 2

| | |
|---|---|
| 1 Mol IPDI | 222,2g |
| 2 Mol Sorbinalkohol | 196,3g |
| 0,01% DBTL | 0,042g |
| 0,2% Jonol CP | 0,839g |
| Aceton | 1000,0g |

Im 500 ml Dreihalskolben wurde Sorbinalkohol aufgeschmolzen (60 °C) und mit DBTL (Dibutylzinndilaurat), Jonol CP und Aceton vorgelegt. Danach wurde auf 40 °C erwärmt. Anschließend wurde IPDI bei 40 °C innerhalb 3h zugetropft. Die Reaktiönsmischung war NCO frei nach 6 h bei 40 °C = 0,67 Gew.-%, es wurde weitere 6 h bei 40 °C gerührt, NCO - Gehalt = 0,12 Gew.-%. Danach wurde das Aceton im Vakuumtrockenschrank bei 40 °C entfernt und der erhaltene Feststoff gemahlen. Smp.: 80 - 85 °C

### Beispiel 2

### VESTANAT T1890 (Trimer des IPDI)-Sorbinalkohol-Addukt

| | |
|---|---|
| 1 Mol T1890/100 | 736,8g |
| 3 Mol Sorbinalkohol | 294,4g |
| 0,01% DBTL | 0,103g |
| 0,2% Jonol CP | 2,06g |
| 50% Aceton | |

Sorbinalkohol, DBTL, Jonol CP wurden in Aceton gelöst (50%ig). Danach wurde IPDI-T1890 50%ig in Aceton gelöst und bei 40 °C innerhalb von 2 h zur Sorbinalkohol-Lösung zugetropft und 16 h nachgerührt, NCO-Gehalt = 0,1 Gew.-%. Das Aceton wurde im Vakuumtrockenschrank bei 40 °C entfernt. Der erhaltene Feststoff wurde gemahlen.

### Diels-Alder-Reaktion

**Materialien** Isophoron-bis-(sorbin-carbamat) (IPDI-SA) (Evonik Industries AG),
1,4-bis(Brommethyl)benzol (97 %, Aldrich),
Natriumhydrid (60% Dispersion in Mineralöl, Aldrich),
Tetrahydrofuran (THF, wasserfrei, ≥ 99,9 %, ABCR),
Diethylphosphat (> 99,0 %, Fluka),
Schwefelkohlenstoff (wasserfrei, ≥ 99,9 %, Aldrich),
Zink 2-ethylhexanoat (97 %, Aldrich) und Acetonitril (wasserfrei, 99,8 %, Fluka) wurden im gelieferten Zustand verwendet.

Zinkchlorid (Aldrich) wurde vakuumgetrocknet und unter Schutzgasatmosphäre gelagert.

Alle anderen Lösemittel wurden ohne weitere Reinigung verwendet.

**Charakterisierungen.** *Die ¹H Kernspinresonanz (NMR)-Spektroskopie* wurde mit einem Bruker AM 250 Spektrometer, der bei 500 MHz betrieben wurde, für Wasserstoffkerne durchgeführt. Alle Proben wurden entweder in CDCl₃ oder DMSO-*d₆* aufgelöst. Die δ-Skala wurde auf den internen Standard Trimethylsilan (TMS, *δ* = 0,00 ppm) kalibriert.

*Gelpermeationschromatographie- (GPC)* Messungen wurden auf einem Polymer Laboratories (Varian) PL -GPC 50 Plus Integrated System, das einen Autosampler, eine PL-Gel-, 5 µm Kugelgröße- Vorsäule (50 × 7,5 mm), eine PL-Gel, 5µm gemischte E-Säule (300 x 7,5 mm), drei PL-Gel-, 5 µm gemischte C-Säulen (300 × 7,5 mm) und einen Differenzial-Brechungsindexdetektor enthielt, mit THF als Eluent bei 35 °C und einer Strömungsrate von 1 mL min⁻¹ durchgeführt. Das SEC-System wurde mit linearen Poly(styrol)standards, die von 160 - 6 × 10⁶ g mol⁻¹ reichten, und linearen Poly(methylmethacrylat)standards, die von 700 - 2 × 10⁶ g mol⁻¹ reichten, kalibriert. Molekulargewichte relativ zu PS sind in der vorliegenden Arbeit angegeben.

*Massenspektrometrie* wurde auf einem IXQ Massenspektrometer (ThermoFisher Scientific) durchgeführt, das mit einer Ionisierungsquelle unter Atmosphärendruck ausgestattet war, die im Zerstäuber-unterstützten Elektrospraymodus arbeitete, der im positiven Ionenmodus verwendet wurde. Das Instrument wurde im *m*/*z* Bereich 195-1822 mit Hilfe eines Standards kalibriert, der Koffein, Met-Arg-Phe-Ala Acetat (MRFA) und ein Gemisch aus fluorinierten Phosphazenen (Ultramark 1621) (Aldrich) enthielt. Proben (c = 0,1 - 0,2 mg mL⁻¹) wurden in einem 3:2 v/v Gemisch aus THF und Methanol, dotiert mit Natriumacetat (0,014 mg mL⁻¹) aufgelöst. Alle Spektren wurden innerhalb des *m*/*z* Bereichs von 150 - 2000 mit einer Sprühspannung von 5 kV und einer Kapillartemperatur von 275°C erhalten. Stickstoff wurde als Schutzgas (Strömung: maximal 45%) verwendet und Helium wurde als Hilfsgas (Strömung: maximal 5%) verwendet. Theoretische Massenberechungen wurden mit der IsotopeViewer Version 1.0 Software durchgeführt.

### Synthese von 1,4-Phenylenbis(methylen)bis((diethoxyphosphoryl)methandithioformat) (P-Di-Linker) (1).

**P**-Di-Linker **1** wurde nach der folgenden Prozedur synthetisiert. Eine Lösung aus Diethylphosphit (5,3 mL, 41,2 mmol) in wasserfreiem THF (20 mL) wurde tropfenweise unter Stickstoff einer Suspension aus NaH (1,64 g, 41,2 mmol) in THF (40 mL) in einem Zweihalskolben zugegeben, der mit einem Rückflusskondensator und einem magnetischen Rührer versehen war. Sobald die Entwicklung von Wasserstoff endete, wurde das Gemisch 10 Minuten unter Rückfluss erwärmt. Nach Abkühlen auf Raumtemperatur wurde das Gemisch in einem flüssigen Stickstoffbad weiter gekühlt. CS₂ (12,26 mL), 203,6 mmol) wurde dann tropfenweise zugegeben und das Gemisch wurde auf Raumtemperatur erwärmen gelassen. Mit dem Rühren wurde weitere 30 min fortgefahren, wonach 1,4-bis(Brommethylbenzol) (5,44 g), aufgelöst in wasserfreiem THF (40 mL) dem Reaktionsgemisch tropfenweise zugegeben wurde. Mit dem Rühren wurde 3 h bei Raumtemperatur fortgefahren und dann wurden 200 mL Hexan zugegeben und das Reaktionsgemisch wurde filtriert. Das violette Filtrat wurde gesammelt und das Lösemittel unter vermindertem Druck entfernt. Das Rohprodukt wurde durch Säulenchromatographie über Silicagel gereinigt, zuerst mit Hexan als Eluent zur Entfernung von Unreinheiten und dann mit Ethylacetat als Eluent zum Sammeln des Produkts **1**. Nach Entfernung des Lösmittels unter verringertem Druck wurde der P-Di-Linker **1** als dunkelfuchsiafarbener Feststoff (60% Ausbeute) erhalten. ¹H NMR (250 MHz, DMSO-*d*₆, 25 °C): δ (ppm) = 7,36 (s, 4H, Ar**H**), 4,57 (s, 4H, -C**H**₂S-), 4,21-4,08 (m, 8H, -OC**H**₂CH₃), 1,30-1,21 (t, *J* = 7 Hz, 12 H,-CH₂C**H**₃). ESI-MS+Na (*m*/*z*) berechnet 553.01; gefunden 553,12.

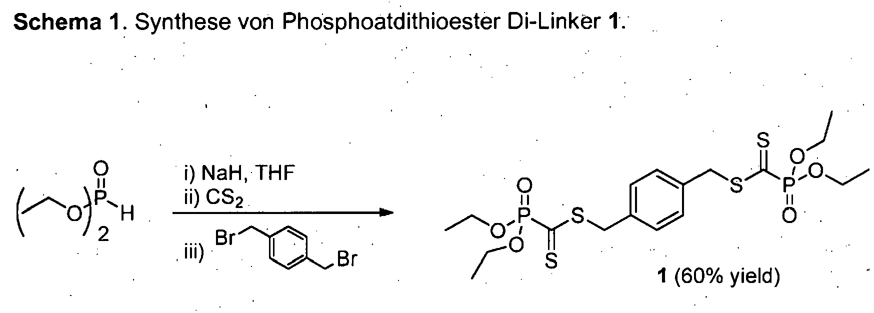

### Diels-Alder-Reaktion : Stufenpolymerisation von P-Di-Linker 1 und IPDI-SA 2, die zu Produkt 3 führt.

Eine typische Polymerisationsprozedur war wie folgt: Sowohl IPDI-SA wie auch P-Di-Linker wurden separat in Acetonitril aufgelöst und in einem Verhältnis von 1 : 1 in Bezug auf funktionelle Gruppen so gemischt, dass die Konzentration der erhaltenen Lösung 1,8 M war. Dem Reaktionsgemisch wurden 1,1 Äquivalente Zinkchlorid (ZnCl₂) zugegeben. Dieses Gemisch wurde 4 h auf 50 °C erwärmt. Das viskose Gemisch wurde in 1 mL Chloroform verdünnt, mit Wasser zur Entfernung von ZnCl₂ extrahiert und getrocknet (85 - 96 % Ausbeute). Diese Stufenpolymerisationsreaktion wurde in Gegenwart von Acetonitril durchgeführt, um das Produkt 3 zu erhalten. Das Produkt wurde durch SEC analysiert. *Mₙ* = 8100 g mol⁻¹ nach 2 Stunden.

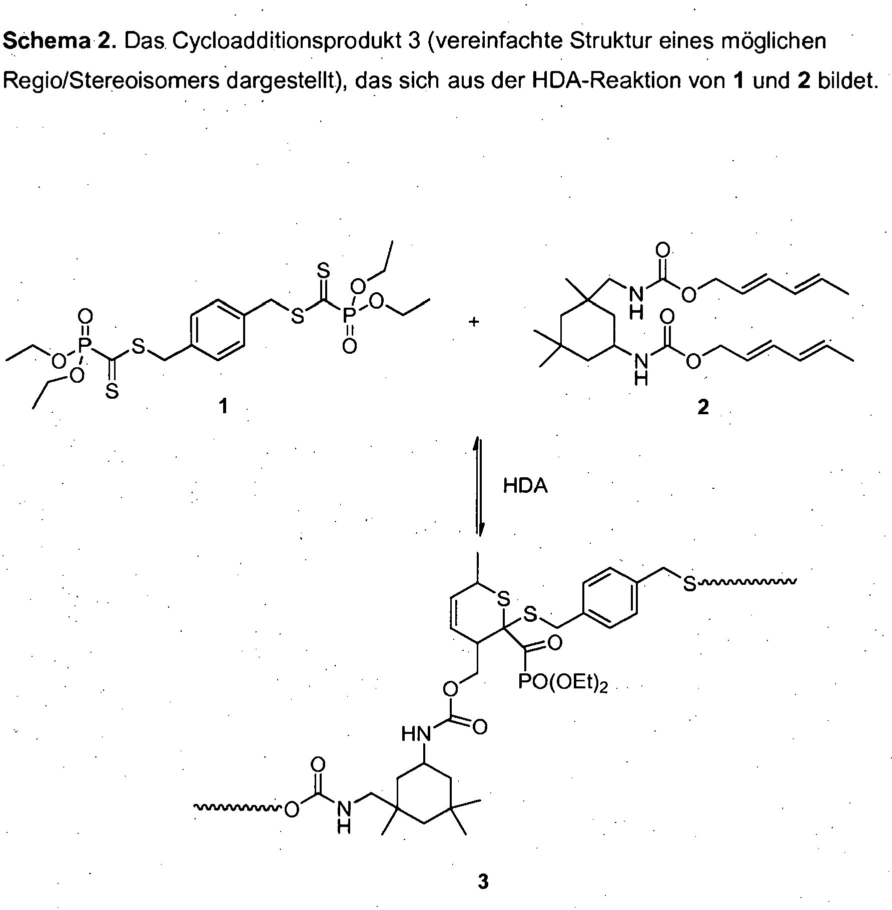

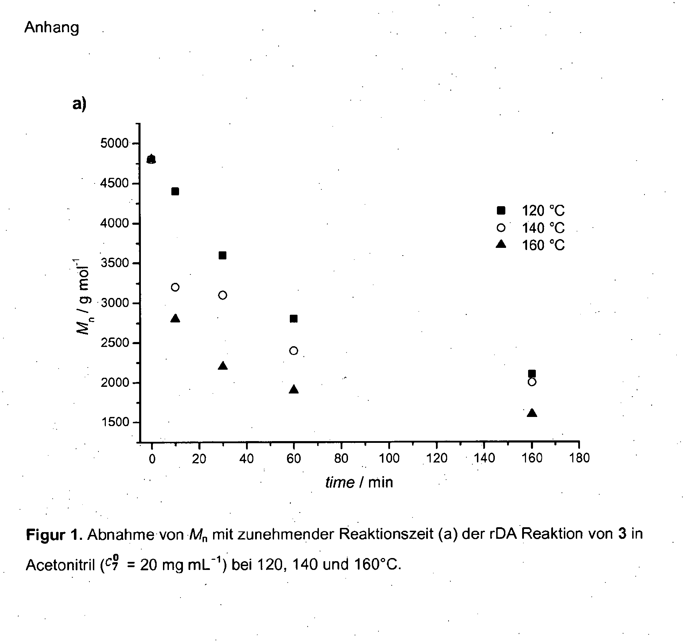

**Retro-Diels-Alder-Reaktion (rDA) von 3**. Eine typische Reaktionsprozedur war wie folgt: 100 mg des Polymers 3 wurden in 5 mL Acetonitril aufgelöst. Unter Rühren wurde die Lösung in einer Druckröhre 40 min auf 140 °C erwärmt. Die Temperatur wurde von 120 - 160 °C variiert und die Reaktionszeit wurde von 10 min bis 160 min variiert. Dann wurde sie rasch mit flüssigem Stickstoff abgeschreckt. Eine Probe (0,1 mL) des rDA-Produkts wurde in THF (0,4 mL) verdünnt und durch GPC und Massenspektometrie analysiert. Die Ergebnisse sind in Figur 1 dargestellt.

## Patentansprüche

1. Reaktionsprodukt aus
A) mindestens einem Isocyanat und/oder Amin mit mindestens zwei funktionellen Gruppen pro Molekül der allgemeinen Formel 1 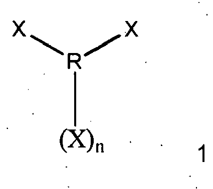 mit der Bedeutung
n= 0, 1,
X= NCO, NH₂, NHA,
A= H, gleichzeitig oder unabhängig voneinander Alkylrest mit 1 bis 16 Kohlenstoffatomen, cyclischer Kohlenwasserstoffrest, aromatischer Kohlenwasserstoffrest, wobei dieser Rest auch Heteroatome und/oder funktionelle Gruppen und/oder Doppelbindungen enthalten kann,
R= aliphatischer oder cycloaliphatischer Kohlenwasserstoffrest, der auch Heteroatome und/oder funktionelle Gruppen und/oder Doppelbindungen enthalten kann,
und
B) mindestens einem Dien mit einer funktionellen Gruppe der allgemeinen Formel 2 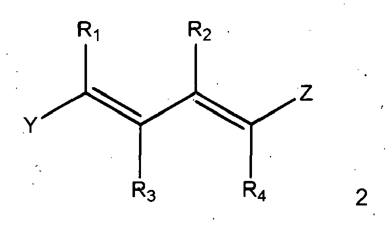 mit der Bedeutung
Y= CH₂OH, COOH, COOA, CH₂NH₂, CH₂NHA,
A, Z und R¹ - R⁴ = gleichzeitig oder unabhängig von einander Alkylrest mit 1 bis 16 Kohlenstoffatomen, cyclischer Kohlenwasserstoffrest, aromatischer Kohlenwasserstoffrest, wobei diese Reste auch Heteroatome und/oder funktionelle Gruppen und/oder Doppelbindungen enthalten können,
wobei alle funktionellen Gruppen X von A) mit der äquivalenten Menge von B) umgesetzt sind.

2. Reaktionsprodukt nach Anspruch 1,
wobei als Komponente A) aliphatischen und cycloaliphatischen Diisocyanate oder Isocyanurate eingesetzt werden.

3. Reaktionsprodukt nach Anspruch 2, wobei IPDI, TMDI, HDI und/oder H12MDI, allein oder in Mischungen und/oder die Trimeren von IPDI, HDI und/oder H₁₂MDI, allein oder in Mischungen eingesetzt werden.

4. Reaktionsprodukt nach Anspruch 1,
wobei als Komponente A) Diamine ausgewählt aus 1,3- und 1,4-Diaminomethylcyclohexan, Hexandiamin-1,6 (HDA), 2,2,4- bzw. 2,4,4-Trimethylhexanamin-1,6 sowie deren Gemische, 3-Aminomethyl-3,5,5-trimethylcyclohexylamin (Isophorondiamin, IPDA), 4,4'-Methylendicyclohexyldiamin, 2,4-Methylendicyclohexyldiamin, 2,2'-Methylendicyclohexyldiamin sowie beliebige Mischungen dieser Isomeren (H₁₂MDA), Polyetherdiamine, eingesetzt werden, es können auch Gemische der Amine eingesetzt werden, bevorzugt IPDA, HDA und/oder H₁₂MDA.

5. Reaktionsprodukt nach mindestens einem der vorherigen Ansprüche, wobei als Komponente B) Sorbinalkohol und/oder Sorbinsäure eingesetzt wird.

6. Reaktionsprodukt nach mindestens einem der vorherigen Ansprüche 1 bis 4, wobei als Komponente B) Retinoide der folgenden Formel 5: 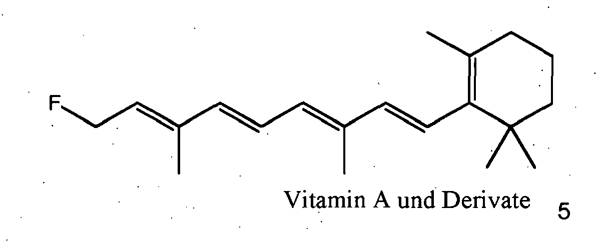 F = OH, COOH, COOK, CHO,
mit K gleich Alkylrest mit 1 bis 6 Kohlenstoffatomen, cyclischer Kohlenwasserstoffrest, aromatischer Kohlenwasserstoffrest, wobei dieser Rest auch Heteroatome und/oder funktionelle Gruppen und/oder Doppelbindungen enthalten kann.

7. Verwendung von Reaktionsprodukten aus
A) mindestens einem Isocyanat und/oder Amin mit mindestens zwei funktionellen Gruppen pro Molekül der allgemeinen Formel 1 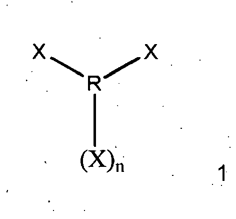 mit der Bedeutung
n = 0, 1,
X = NCO, NH₂, NHA,
A = H, gleichzeitig oder unabhängig voneinander Alkylrest mit 1 bis 16 Kohlenstoffatomen, cyclischer Kohlenwasserstoffrest, aromatischer Kohlenwasserstoffrest, wobei dieser Rest auch Heteroatome und/oder funktionelle Gruppen und/oder Doppelbindungen enthalten kann,
R = aliphatischer oder cycloaliphatischer Kohlenwasserstoffrest, der auch Heteroatome und/oder funktionelle Gruppen und/öder Doppelbindungen enthalten kann,
und
B) mindestens einem Dien mit einer funktionellen Gruppe der allgemeinen Formel 2 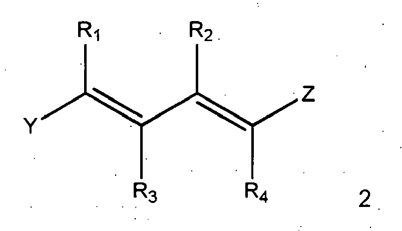 mit der Bedeutung
Y= CH₂OH, COOH, COOA, CH₂NH₂, CH₂NHA,
A, Z und R¹ - R⁴ = gleichzeitig oder unabhängig von einander Alkylrest mit 1 bis 16 Kohlenstoffatomen, cyclischer Kohlenwasserstoffrest, aromatischer Kohlenwasserstoffrest, wobei diese Reste auch Heteroatome und/oder funktionelle Gruppen und/oder Doppelbindungen enthalten können,
wobei alle funktionellen Gruppen X von A) mit der äquivalenten Menge von B) umgesetzt sind,
zur reversiblen oder permanenten Vernetzung mit Verbindungen mit mindestens einer reaktiven Doppelbindung (Dienophile).

8. Verwendung von Reaktionsprodukten nach Anspruch 7, wobei als Dienophil-Komponente ein mittels Atomtransfer radikalische Polymerisation (ATRP) hergestelltes, bifunktionelles Polymer eingesetzt wird.

9. Verwendung von Reaktionsprodukten nach Anspruch 7, wobei als Dienophil-Komponente eine Verbindung mit einer Kohlenstoff-Schwefel-Doppelbindung eingesetzt wird.

10. Verwendung von Reaktionsprodukten nach Anspruch 7, wobei als Dienophil-Komponente ein Dithioester eingesetzt wird.

11. Verwendung von Reaktionsprodukten nach Anspruch 7, wobei als Dienophil-Komponente Verbindung mit der Struktur 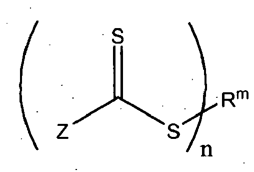 wobei es sich bei Z um eine Elektronen-ziehende Gruppe, bei R^{m} um eine mehrbindige organische Gruppe handelt, n ist eine Zahl zwischen 2 und 20.

12. Verwendung von Reaktionsprodukten nach Anspruch 11, wobei R^{m} bedeutet: eine mehrbindige organische Gruppe, basierend auf verzweigten oder linearen alkylischen, aromatischen oder einer Kombination aus alkylischen und aromatischen multifunktionalen Alkoholen, multifunktionalen halogenierten Verbindungen, multifunktionalen Carbonsäuren oder multifunktionalen Amine, oder ein Polymer.

13. Verwendung von Reaktionsprodukten nach Anspruch 11 bis 12, wobei Z eine 2-Pyridylgruppe, eine Phosphorylgruppe, eine Sulfphonylgruppe, Cyano-oder Trifluoromethylgruppe, darstellt.

14. Verwendung von Reaktionsprodukten nach Anspruch 7 bis13, für Klebstoffe, Formmassen, Tinten, Dichtstoffen, Beschichtungsmassen, Lacke, Farbe, Verbundmaterialien.

15. Verwendung von Reaktionsprodukten nach Anspruch 7 bis 13 bei der Herstellung von Formkörpern z. B. über Rapid-Prototyping-Verfahren.

16. Verwendung von Reaktionsprodukten nach Anspruch 7 bis 15, wobei als Komponente B) Sorbinalkohol und/oder Sorbinsäure eingesetzt wird.

17. Verwendung von Reaktionsprodukten nach Anspruch 7 bis 15, wobei als Komponente B) Retinoide der folgenden Formel 5: 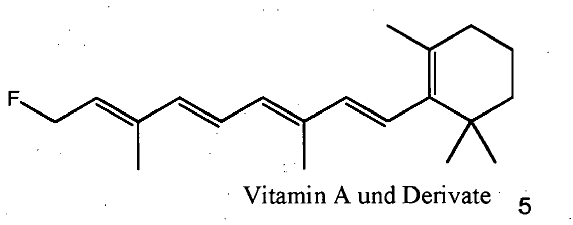 F = OH, COOH, COOK, CHO,
mit K gleich Alkylrest mit 1 bis 6 Kohlenstoffatomen, cyclischer Kohlenwasserstoffrest, aromatischer Kohlenwasserstoffrest, wobei dieser Rest auch Heteroatome und/oder funktionelle Gruppen und/oder Doppelbindungen enthalten kann.

## Claims

1. Reaction product of
A) at least one isocyanate and/or amine having at least two functional groups per molecule of the general formula 1 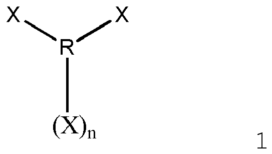 with the following definitions:
n = 0 or 1,
X = NCO, NH₂ or NHA,
A = H, simultaneously or independently of one another alkyl radical having 1 to 16 carbon atoms, cyclic hydrocarbon radical or aromatic hydrocarbon radical, it also being possible for this radical to contain heteroatoms and/or functional groups and/or double bonds,
R = aliphatic or cycloaliphatic hydrocarbon radical, which may also contain heteroatoms and/or functional groups and/or double bonds,
and
B) at least one diene having a functional group of
the general formula 2 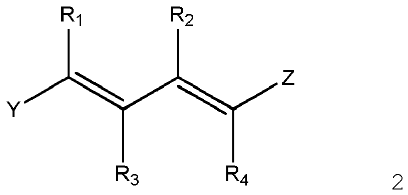 with the following definitions:
Y = CH₂OH, COOH, COOA, CH₂NH₂ or CH₂NHA and
A, Z and R¹ - R⁴ = simultaneously or independently of one another alkyl radical having 1 to 16 carbon atoms, cyclic hydrocarbon radical or aromatic hydrocarbon radical, it also being possible for these radicals to contain heteroatoms and/or functional groups and/or double bonds,
where all of the functional groups X of A) have undergone reaction with the equivalent amount of B).

2. Reaction product according to Claim 1,
where aliphatic and cycloaliphatic diisocyanates or isocyanurates are used as component A).

3. Reaction product according to Claim 2, where IPDI, TMDI, HDI and/or H₁₂MDI, alone or in mixtures, and/or the trimers of IPDI, HDI and/or H₁₂MDI, alone or in mixtures, are used.

4. Reaction product according to Claim 1,
where diamines selected from 1,3- and 1,4-diaminomethylcyclohexane, hexane-1,6-diamine (HDA), 2,2,4- and/or 2,4,4-trimethylhexane-1,6-diamine and also mixtures thereof, 3-aminomethyl-3,5,5-trimethylcyclohexylamine (isophoronediamine, IPDA), 4,4'-methylenedicyclohexyldiamine, 2,4-methylenedicyclohexyldiamine, 2,2'-methylene-dicyclohexyldiamine and also any desired mixtures of these isomers (H₁₂MDA) and polyetherdiamines are used as component A); it is also possible to use mixtures of the amines, preferably IPDA, HDA and/or H₁₂MDA.

5. Reaction product according to at least one of the preceding claims, where sorbic alcohol and/or sorbic acid is used as component B).

6. Reaction product according to at least one of preceding Claims 1 to 4, where retinoids of the following formula 5: 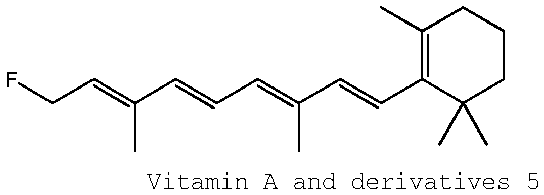 F = OH, COOH, COOK or CHO,
where K is alkyl radical having 1 to 6 carbon atoms, cyclic hydrocarbon radical or aromatic hydrocarbon radical, it also being possible for this radical to contain heteroatoms and/or functional groups and/or double bonds,
are used as component B).

7. Use of reaction products of
A) at least one isocyanate and/or amine having at least two functional groups per molecule of the general formula 1 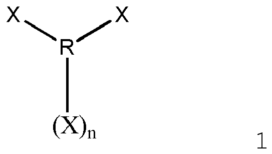 with the following definitions:
n = 0 or 1,
X = NCO, NH₂ or NHA,
A = H, simultaneously or independently of one another alkyl radical having 1 to 16 carbon atoms, cyclic hydrocarbon radical or aromatic hydrocarbon radical, it also being possible for this radical to contain heteroatoms and/or functional groups and/or double bonds,
R = aliphatic or cycloaliphatic hydrocarbon radical, which may also contain heteroatoms and/or functional groups and/or double bonds,
and
B) at least one diene having a functional group of the general formula 2 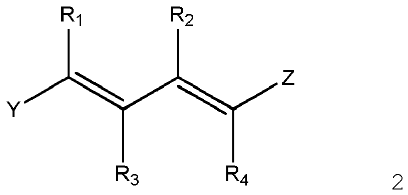 with the following definitions:
Y = CH₂OH, COOH, COOA, CH₂NH₂ or CH₂NHA and
A, Z and R¹ - R⁴ = simultaneously or independently of one another alkyl radical having 1 to 16 carbon atoms, cyclic hydrocarbon radical or aromatic hydrocarbon radical, it also being possible for these radicals to contain heteroatoms and/or functional groups and/or double bonds,
where all of the functional groups X of A) have undergone reaction with the equivalent amount of B),
for reversible or permanent crosslinking with compounds having at least one reactive double bond (dienophiles).

8. Use of reaction products according to Claim 7, where as dienophile component a difunctional polymer prepared by atom transfer radical polymerization (ATRP) is used.

9. Use of reaction products according to Claim 7, where as dienophile component a compound having a carbon-sulphur double bond is used.

10. Use of reaction products according to Claim 7, where as dienophile component a dithioester is used.

11. Use of reaction products according to Claim 7, where as dienophile component a compound having the structure 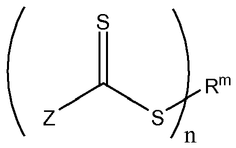 where Z is an electron-withdrawing group, R^{m} is a polyvalent organic group, n is a number between 2 and 20, is used.

12. Use of reaction products according to Claim 11, where R^{m} is a polyvalent organic group based on branched or linear alkylic, aromatic or a combination of alkylic and aromatic polyfunctional alcohols, polyfunctional halogenated compounds, polyfunctional carboxylic acids or polyfunctional amines, or a polymer.

13. Use of reaction products according to Claims 11 to 12, where Z is a 2-pyridyl group, a phosphoryl group, a sulphonyl group, cyano group or trifluoromethyl group.

14. Use of reaction products according to Claims 7 to 13 for adhesives, moulding compounds, inks, sealants, coating materials, varnishes, paints or composite materials.

15. Use of reaction products according to Claims 7 to 13 in the production of mouldings for example via rapid prototyping methods.

16. Use of reaction products according to Claims 7 to 15, where sorbic alcohol and/or sorbic acid is used as component B).

17. Use of reaction products according to Claims 7 to 15, where retinoids of the following formula 5: 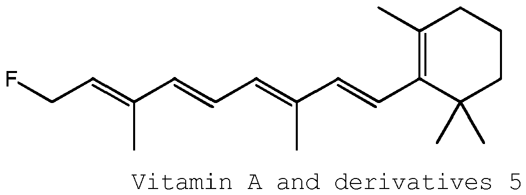 F = OH, COOH, COOK or CHO,
where K is alkyl radical having 1 to 6 carbon atoms, cyclic hydrocarbon radical or aromatic hydrocarbon radical, it also being possible for this radical to contain heteroatoms and/or functional groups and/or double bonds,
are used as component B).

## Revendications

1. Produit de réaction
A) d'au moins un isocyanate et/ou une amine présentant au moins deux groupes fonctionnels par molécule de formule générale 1 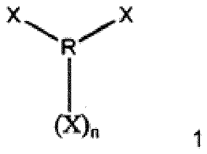 où
n = 0, 1,
X = NCO, NH₂ ; NHA,
A = H, simultanément ou indépendamment l'un de l'autre, un radical alkyle comprenant 1 à 16 atomes de carbone, un radical hydrocarboné cyclique, un radical hydrocarboné aromatique, ce radical pouvant également contenir des hétéroatomes et/ou des groupes fonctionnels et/ou des doubles liaisons,
R = un radical hydrocarboné aliphatique ou cycloaliphatique, qui peut également contenir des hétéroatomes et/ou des groupes fonctionnels et/ou des doubles liaisons,
et
B) d'au moins un diène présentant un groupe fonctionnel de formule générale 2 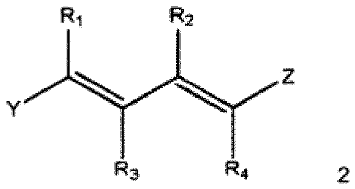 où
Y = CH₂OH, COOH, COOA, CH₂NH₂, CH₂NHA,
A, Z et R¹-R⁴ = simultanément ou indépendamment les uns des autres, un radical alkyle comprenant 1 à 16 atomes de carbone, un radical hydrocarboné cyclique, un radical hydrocarboné aromatique, ces radicaux pouvant également contenir des hétéroatomes et/ou des groupes fonctionnels et/ou des doubles liaisons,
tous les groupes fonctionnels X de A) étant transformés avec la quantité équivalente de B).

2. Produit de réaction selon la revendication 1, des diisocyanates ou des isocyanurates aliphatiques et cycloaliphatiques étant utilisés comme composant A).

3. Produit de réaction selon la revendication 2, de l'IPDI, du TMDI, de l'HDI et/ou de l'H₁₂MDI étant utilisés seuls ou en mélange et/ou les trimères d'IPDI, d'HDI et/ou d'H₁₂MDI étant utilisés seuls ou en mélange.

4. Produit de réaction selon la revendication 1, des diamines choisies parmi le 1,3-diaminométhylcyclohexane et le 1,4-diaminométhylcyclohexane, l'hexanediamine-1,6 (HDA), la 2,2,4-triméthylhexandiamine-1,6 ou la 2,4,4-triméthylhexandiamine-1,6 ainsi que leurs mélanges, la 3-aminométhyl-3,5,5-triméthylcyclohexylamine (isophoronediamine, IPDA), la 4,4'-méthylènedicyclohexyldiamine, la 2,4-méthylènedicyclohexyldiamine, la 2,2'-méthylènedicyclohexyldiamine ainsi que des mélanges quelconques de ces isomères (H₁₂MDA), des polyétherdiamines, étant utilisées comme composant A), des mélanges des amines pouvant également être utilisés, de préférence l'IPDA, l'HDA et/ou l'H₁₂MDA.

5. Produit de réaction selon au moins l'une quelconque des revendications précédentes, de l'alcool sorbique et/ou de l'acide sorbique étant utilisé(s) comme composant B).

6. Produit de réaction selon au moins l'une quelconque des revendications précédentes 1 à 4, des rétinoïdes de la formule 5 suivante étant utilisés comme composant B) : 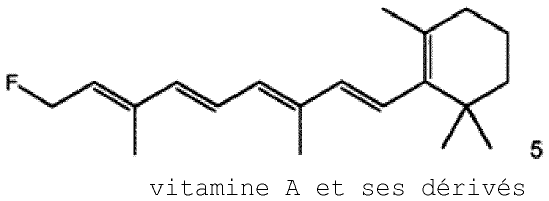 F = OH, COOH, COOK, CHO,
K représentant un radical alkyle comprenant 1 à 6 atomes de carbone, un radical hydrocarboné cyclique, un radical hydrocarboné aromatique, ce radical pouvant également contenir des hétéroatomes et/ou des groupes fonctionnels et/ou des doubles liaisons.

7. Utilisation de produits de réaction
A) d'au moins un isocyanate et/ou une amine présentant au moins deux groupes fonctionnels par molécule de formule générale 1 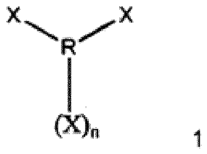 où
n = 0, 1,
X = NCO, NH₂, NHA,
A = H, simultanément ou indépendamment l'un de l'autre, un radical alkyle comprenant 1 à 16 atomes de carbone, un radical hydrocarboné cyclique, un radical hydrocarboné aromatique, ce radical pouvant également contenir des hétéroatomes et/ou des groupes fonctionnels et/ou des doubles liaisons,
R = un radical hydrocarboné aliphatique ou cycloaliphatique, qui peut également contenir des hétéroatomes et/ou des groupes fonctionnels et/ou des doubles liaisons,
et
B) d'au moins un diène présentant un groupe fonctionnel de formule générale 2 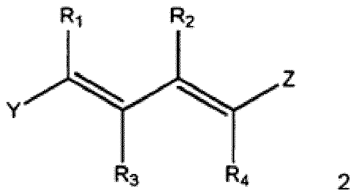 où
Y = CH₂OH, COOH, COOA, CH₂NH₂, CH₂NHA,
A, Z et R¹-R⁴ = simultanément ou indépendamment les uns des autres, un radical alkyle comprenant 1 à 16 atomes de carbone, un radical hydrocarboné cyclique, un radical hydrocarboné aromatique, ces radicaux pouvant également contenir des hétéroatomes et/ou des groupes fonctionnels et/ou des doubles liaisons,
tous les groupes fonctionnels X de A) étant transformés avec la quantité équivalente de B), pour la réticulation réversible ou permanente avec des composés présentant au moins une double liaison réactive (diénophiles).

8. Utilisation de produits de réaction selon la revendication 7, au moins un polymère difonctionnel préparé par polymérisation radicalaire par transfert d'atomes (ATRP) étant utilisé comme composant diénophile.

9. Utilisation de produits de réaction selon la revendication 7, un composé présentant une double liaison carbone-soufre étant utilisé comme composant diénophile.

10. Utilisation de produits de réaction selon la revendication 7, un dithioester étant utilisé comme composant diénophile.

11. Utilisation de produits de réaction selon la revendication 7, un composé présentant la structure 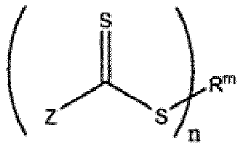 étant utilisé comme composant diénophile, Z étant un groupe attracteur d'électrons, R^{m} étant un groupe organique polyvalent, n valant un nombre entre 2 et 20.

12. Utilisation de produits de réaction selon la revendication 11, R^{m} signifiant : un groupe organique polyvalent basé sur des alcools polyvalents, ramifiés ou linéaires, alkyliques, aromatiques ou une combinaison d'alcools polyvalents alkyliques et aromatiques, des composés halogénés polyfonctionnels, des acides carboxyliques polyfonctionnels ou des amines polyfonctionnelles ou un polymère.

13. Utilisation de produits de réaction selon la revendication 11 à 12, Z représentant un groupe 2-pyridyle, un groupe phosphoryle, un groupe sulfonyle, un groupe cyano ou un groupe trifluorométhyle.

14. Utilisation de produits de réaction selon la revendication 7 à 13 pour des adhésifs, des masses de moulage, des encres, des substances d'étanchéité, des masses de revêtement, des laques, des peintures, des matériaux composites.

15. Utilisation de produits de réaction selon la revendication 7 à 13 lors de la fabrication de corps façonnés, par exemple via un procédé de prototypage rapide.

16. Utilisation de produits de réaction selon la revendication 7 à 15, de l'alcool sorbique et/ou de l'acide sorbique étant utilisé comme composant B).

17. Utilisation de produits de réaction selon la revendication 7 à 15, des rétinoïdes de la formule 5 suivante étant utilisés comme composant B) : 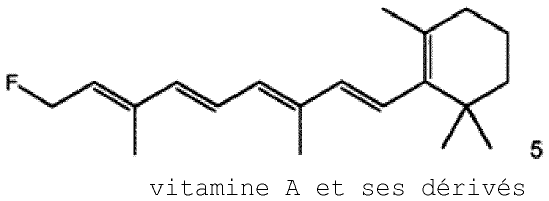 F = OH, COOH, COOK, CHO,
K représentant un radical alkyle comprenant 1 à 6 atomes de carbone, un radical hydrocarboné cyclique, un radical hydrocarboné aromatique, ce radical pouvant également contenir des hétéroatomes et/ou des groupes fonctionnels et/ou des doubles liaisons.
